# EUROPEAN PATENT SPECIFICATION

(11) **EP 2 747 674 B1**
(45) Date of publication and mention of the grant of the patent: **09.12.2015**
(21) Application number: 12815829.2
(22) Date of filing: 12.12.2012
(51) Int. Cl.: A61B 17/22, A61N 7/02

(54) **CALCULATING THE ULTRASONIC INTENSITY ESTIMATE USING AN INCOHERENT SUM OF THE ULTRASONIC PRESSURE GENERATED BY MULTIPLE TRANSDUCER ELEMENTS**
KALKULATION DER ULTRASCHALLINTENSITÄT MITTELS INKOHÄRENTER SUMME DES VON MEHREREN WANDLERELEMENTEN ERZEUGTEN ULTRASCHALLDRUCKS
CALCUL DE L'ESTIMATION D'INTENSITÉ ULTRASONORE À L'AIDE D'UNE SOMME INCOHÉRENTE DE LA PRESSION ULTRASONORE GÉNÉRÉE PAR DE MULTIPLES ÉLÉMENTS DE TRANSDUCTEUR

(30) Priority: 22.12.2011 US 201161579127 P; 22.12.2011 EP 11195121
(43) Date of publication of application: 02.07.2014
(73) Proprietor: Koninklijke Philips N.V., 5656 AE Eindhoven (NL)
(72) Inventor: KOSKELA, Ilpo, Asko, Julius, 5656 AE Eindhoven (NL); MOUGENOT, Charles, 5656 AE Eindhoven (NL); HÄKKINEN, Marko, Tapani, 5656 AE Eindhoven (NL)
(74) Representative: Steffen, Thomas
(86) International application number: PCT/IB2012/057204
(87) International publication number: WO 2013/093716

(56) References cited:
- US-A1- 2009 230 823
- US-A1- 2010 222 676
- US-A1- 2011 306 881
- US-B2- 7 699 780

## Description

### TECHNICAL FIELD

The invention relates to high intensity focused ultrasound, in particular to the estimation of ultrasonic intensities by the use of an incoherent sum of the ultrasonic pressure generated by multiple transducer elements.

### BACKGROUND OF THE INVENTION

Ultrasound is quickly becoming a desired approach for specific therapeutic interventions. In particular, the use of high intensity focused ultrasound is currently being used as an approach for thermal therapeutic intervention for uterine fibroids and has been examined for possible uses in the treatment of the liver, the brain, and the prostate. Ultrasound therapy for tissue ablation works by sonicating a tissue of interest with high intensity ultrasound that is absorbed and converted into heat, raising the temperature of the tissues. As the temperature rises coagulative necrosis of the tissues may occurs resulting in immediate cell death. The transducers used in therapy can be outside the body or be inserted into the body; e.g., through blood vessels, urethra, rectum, and etc.

In high intensity focused ultrasound an array of transducer elements are used to form an ultrasonic transducer. Supplying alternating current electrical power to the transducer elements causes them to generate ultrasonic waves. The ultrasonic waves from each of the transducer elements either add constructively or destructively. By controlling the phase of alternating current electrical power supplied to each of the transducer elements the focal point or target volume into which the ultrasound power is focused may be controlled.

Along the path of the ultrasound from individual transducer elements to the focal point the ultrasound can also add constructively and destructively. This can lead to hot spots or regions which are unintentionally heated or sonicated. There is therefore the risk that sensitive anatomical regions can be unintentionally injured during a sonication.

United States patent US 7,699,780 B2 describes a method of delivering ultrasound energy towards a target tissue from transducer elements such that the energy intensity in a target is at or above a prescribed treatment level. Additionally, the energy intensity in a tissue region to be protected within the ultrasound energy path is at or below a prescribed safety level. Further, the US-patent US 7 699 780 discloses delivering ultrasound energy towards the target tissue, while the energy intensity at tissue to be protected is below a predetermined safety level. The operation criteria for the transducer elements are determined by way of a ray model.

### SUMMARY OF THE INVENTION

The invention provides for a medical instrument, a computer program product, and a method of operating the medical instrument in the independent claims. Embodiments are given in the dependent claims.

It may be difficult to accurately predict the exact location of an unintentional heating zone along the ultrasonic path to the focal point. One difficulty is that the phases add constructively and destructively. This may make it computationally intensive to calculate the estimate. Another difficulty is that the accuracy of the prediction is limited by the accuracy of the model used. Within a living organism there are different tissue types and errors in the model may result in errors in predicting where unintentional heating zones are.

Embodiments of the invention may address these and other problems by estimating the ultrasonic heating in a protected zone by using an incoherent sum of the ultrasonic pressures from the individual ultrasonic transducer elements. This may have the advantage that the calculation is faster. The ultrasonic pressure field created by each individual transducer element can be calculated. The overall pressure at each location is estimated by the sum of the squares of the individual pressures. Another advantage is that using the incoherent sum may be effective in predicting the possible locations of unintentional heating zones. In the incoherent sum the constructive and destructive adding of the pressure is ignored. The result is that the incoherent sum may be useful for identifying regions which may have unintentional heating. This may reduce the likely hood of an error caused by an inaccurate model. This reduction in the likelihood of an error may be safer than a system which uses a coherent sum of the pressures to predict the location of unintentional heating zones.

A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, punched tape, punch cards, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. References to a computer-readable storage medium should be interpreted as possibly being multiple computer-readable storage mediums. Various executable components of a program or programs may be stored in different locations. The computer-readable storage medium may for instance be multiple computer-readable storage medium within the same computer system. The computer-readable storage medium may also be computer-readable storage medium distributed amongst multiple computer systems or computing devices.

'Computer memory' or 'memory' is an example of a computer-readable storage medium. Computer memory is any memory which is directly accessible to a processor. Examples of computer memory include, but are not limited to: RAM memory, registers, and register files. References to 'computer memory' or 'memory' should be interpreted as possibly being multiple memories. The memory may for instance be multiple memories within the same computer system. the memory may also be multiple memories distributed amongst multiple computer systems or computing devices.

'Computer storage' or 'storage' is an example of a computer-readable storage medium. Computer storage is any non-volatile computer-readable storage medium. Examples of computer storage include, but are not limited to: a hard disk drive, a USB thumb drive, a floppy drive, a smart card, a DVD, a CD-ROM, and a solid state hard drive. In some embodiments computer storage may also be computer memory or vice versa. References to 'computer storage' or 'storage' should be interpreted as possibly being multiple storage devices. The storage may for instance be multiple storage devices within the same computer system or computing device. The storage may also be multiple storages distributed amongst multiple computer systems or computing devices.

A 'processor' as used herein encompasses an electronic component which is able to execute a program or machine executable instruction. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. Many programs have their instructions performed by multiple processors that may be within the same computing device or which may even be distributed across multiple computing devices.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, one or more switches, one or more buttons, and accelerometer are all examples of user interface components which enable the receiving of information or data from an operator.

A 'hardware interface' as used herein encompasses a interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical image data is defined herein as two or three dimensional data that has been acquired using a medical imaging scanner. A medical imaging scanner is defined herein as a apparatus adapted for acquiring information about the physical structure of a patient and construct sets of two dimensional or three dimensional medical image data. Medical image data can be used to construct visualizations which are useful for diagnosis by a physician. This visualization can be performed using a computer.

Magnetic Resonance (MR) data is defined herein as being the recorded measurements of radio frequency signals emitted by atomic spins by the antenna of a Magnetic resonance apparatus during a magnetic resonance imaging scan. A Magnetic Resonance Imaging (MRI) image is defined herein as being the reconstructed two or three dimensional visualization of anatomic data contained within the magnetic resonance imaging data. This visualization can be performed using a computer.

An 'ultrasound window' as used herein encompasses a window which is able to transmit ultrasonic waves or energy. Typically a thin film or membrane is used as an ultrasound window. The ultrasound window may for example be made of a thin membrane of BoPET (Biaxially-oriented polyethylene terephthalate).

In one aspect the invention provides for a medical instrument comprising a high-intensity focused ultrasound system comprising an ultrasonic transducer. The ultrasonic transducer comprises multiple transducer elements. The high-intensity focused ultrasound system is operable for switching on and off the supply of electrical power to each of the multiple transducer elements. The medical instrument further comprises a processor for controlling the medical instrument. The medical instrument further comprises a memory containing machine-executable instructions. Execution of the instructions causes the processor to receive a treatment plan specifying a protected zone within a subject. Execution of the instructions further cause the processor to calculate a set of transducer control parameters using the treatment plan such that ultrasonic intensity estimate in the protected zone is below a predetermined threshold.

The set of transducer control parameters specify the switching of electrical power to each of the multiple transducer elements. That is to say the set of transducer control parameters may be used to switch on and off individual or groups of transducers. An ultrasonic intensity estimate may be calculated in this step. The ultrasonic intensity estimate is calculated using an incoherent sum of the ultrasonic pressure generated by each of the multiple transducer elements. This embodiment may be beneficial because the use of an incoherent sum makes it easier and less computationally intensive to calculate the ultrasonic intensity estimate. This makes it more feasible to calculate the set of transducer control parameters.

As used herein an incoherent sum of the ultrasonic pressure generated by each of the multiple transducer elements encompasses calculating the ultrasonic pressure generated by each of the multiple transducer elements and squaring it then adding the values together. This sum is labeled the incoherent sum because the phases are not taken into consideration. It has the benefit of producing a good estimate of the maximum ultrasonic intensity which can be generated within the protected zone. It is however much computationally less intensive than calculating a value which takes into account the coherent sum or the phases. Also there may be insufficient knowledge of the internal anatomy of the subject or the ultrasonic properties of the internal anatomy. Using the incoherent sum eliminates the possibility that a hot spot would be generated by the ultrasound and incorrectly calculated as being a point of low ultrasonic intensity. Using the incoherent sum may produce an estimate of the ultrasonic intensity which can be reasonably relied upon in a clinical setting.

The electrical power supplied to the multiple transducer elements may be alternating current electrical power or it may be pulsed electrical power. The transducer elements typically use a piezoelectric or other actuator which responds to a control voltage or current.

In another embodiment the multiple transducer elements may be turned off individually or may be turned off in groups of transducer elements.

In another embodiment the treatment plan can specify the geometry and internal structure of the subject. For instance the treatment plan may include medical image data which is registered to the high-intensity focused ultrasound system. In other embodiments the treatment plan may contain a plan which is constructed by a physician and may be possibly constructed using unregistered medical image data. The treatment plan may also include anatomical landmarks which may be used to register medical image data to the treatment plan.

In another embodiment execution of the instructions further causes the processor to sonicate the target zone using at least partially the set of transducer control parameters. For instance the set of transducer control parameters may be used in conjunction with the treatment plan to generate a set of high-intensity focused ultrasound system controls which may be used to control the high-intensity focused ultrasound system to sonicate a target zone of the subject.

In some embodiments the target zone may be a path. A path as used herein may be a set of individual sonication locations that are sequentially sonicated by the high-intensity focused ultrasound system. The target or path of the subject and also the location of the protected zone may be time-dependent. For this reason the set of transducer control parameters may also be therefore time-dependent.

In another embodiment the incoherent sum of the ultrasonic pressure generated by each of the multiple transducer elements is multiplied by a coherence factor to calculate the ultrasonic intensity estimate. The coherence factor is a factor which is used to compensate for the fact that the incoherent sum ignores the individual phases of the ultrasound generated by each of the multiple transducer elements. The use of the coherence factor may be beneficial because it allows a safety margin to be introduced. An equivalent embodiment would be to lower the predetermined threshold such that the threshold is lower to take into account the same safety considerations.

In another embodiment the coherence factor is spatially-dependent. This may be beneficial because the difference between incoherent sum and the hot spots in the coherent sum based on the phase of the multiple transducer elements increases towards the focus, and may be different depending upon the spatial location within the subject.

In another embodiment the coherence factor may be predetermined or pre-calculated for a particular transducer. For instance for a particular transducer the coherence factor which is spatially-dependent may be calculated from a calculated coherence sum, that is calculations may be done in advance using the phases and used to generate the coherence factor.

In another embodiment the coherence factor is determined for different types of tissues. For instance the affect of adipose, muscle and other tissues may be taken into account and may be predetermined or calculated.

In another embodiment the coherence factor may depend on the trajectory to be sonicated. For instance the ultrasonic transducer may have an electronically adjustable focus and the focus may be adjusted by individually controlling the phase of electrical power to each of the multiple transducer elements. For a particular trajectory the coherence factor may be pre-calculated. In some embodiments this may even be included in the treatment plan.

In another embodiment the medical instrument further comprises a medical imaging system for acquiring medical image data within an imaging zone. Execution of the instructions further causes the processor to acquire the medical image data. The protected zone is within the imaging zone. The set of transducer control parameters are calculated at least partially using the medical image data. This embodiment may be beneficial because the actual tissue type or internal structure of the subject may be taken into account when calculating which of the multiple transducer elements to switch on and off. For instance in some embodiments this may have the benefit because the medical image data can be registered to the treatment plan and the location of the protected zone may be identified. This may enable the correct switching on and off of the transducer elements. In other embodiments this may enable the coherence factor to be calculated more accurately.

In another embodiment execution of the instructions further cause the processor to calculate an image segmentation using the medical image data. The image segmentation identifies tissue types within the subject. Execution of the instructions further cause the processor to calculate the coherence factor at least partially using the image segmentation. This may be beneficial because within different tissue types the ultrasound may travel at different speeds. This may enable the more accurate calculation of the coherence factor.

In some embodiments both the calculation of an image segmentation and/or specifying which of the set of transducer control parameters may be modified for instance using input received from a user. This may be received via a user interface or from a different computer program.

In another embodiment the medical imaging system is a computer tomography system.

In another embodiment the medical imaging system is a magnetic resonance imaging system.

In another embodiment the medical imaging system is a diagnostic ultrasound system.

In another embodiment the coherence factor is calculated at least partially using a coherent sum of the ultrasonic pressure generated by each of the multiple transducer elements. In this embodiment the pressure taking into account of the phase are added for each location or voxel or cell and then is squared to find the intensity. This has the advantage that the coherence factor which is calculated has taken into account the phase of the individual components of the ultrasound from each of the multiple transducer elements. This may have the possibility of leading to a more accurate coherence factor.

In another embodiment the ultrasound transducer has an electronically adjustable focus. The high-intensity focused ultrasound system is operable for controlling the electronically adjustable focus by controlling the phase of electrical power to each of the multiple transducer elements. The target zone is a path. The electronically adjustable focus is used to focus the ultrasonic energy to a target zone. A path as used herein is a collection or set of sonication locations which are sequentially sonicated. Execution of the instructions further causes the processor to calculate a set of time-dependent controlling phases. The time-dependent controlling phases specify the phase of electrical power applied to each of the multiple transducer elements as a function of time such that the electronically adjustable focus follows the path. In other words, the ultrasound is focused at different locations along the path as a function of time.

Execution of the instructions further cause the processor to calculate the coherence sum at least partially using the set of time-dependent controlling phases. This may be beneficial because the relative phase of the multiple transducer elements may not take all possible values if a particular trajectory is followed with the focus of the ultrasonic transducer. This may enable a more accurate calculation of the coherence factor particularly a spatially-dependent coherence factor.

In another embodiment the set of transducer control parameters further comprise any one of the following: phase of electrical power supplied to each of the multiple transducer elements, amplitude of the electrical power supplied to each of the multiple transducer elements, power level of the electrical power supplied to each of the multiple transducer elements, alternating frequency of electrical power to each of the multiple transducer elements, duration of electrical power supplied to each of the multiple transducer elements, the sonication trajectory, and combinations thereof. In some embodiments one or more of these parameters may be controlled individually or in groups. Some may be controlled individually and some may be controlled in groups. The high-intensity focused ultrasound system may be operable for controlling each of these additional parameters. In addition some or all of the transducer control parameters may be time-dependent. That is to say the value of the transducer control parameters may change as a function of time.

In another embodiment the set of transducer element parameters is calculated by simulating the switching on and off of combinations of the multiple transducer elements. Various combinations of transducer elements being active or deactivated may be tried to see which may be used to most efficiently heat a target zone and in addition protect the protected zone. For instance it may be possible to select channels to turn off using an iterative algorithm. One or several elements to turn off may be selected based on an incoherent sum once a corresponding simulation is done to decide if additional elements should be turned off and/or to upload coherence factor.

In another embodiment the set of phases is calculated by solving a combinatorial optimization problem.

In another embodiment execution of the instructions further cause the processor to model at least the protected zone as multiple regions. The set of transducer element states are solved using a linear programming problem for the multiple regions. Essentially the protected zone may be divided into individual cells or voxels and the various values of the different parameters such as the coherence factor may be calculated or estimated for that individual cell or voxel. The use of the linear programming may be beneficial because it allows for very rapid solving of the incoherence sum. This may even enable real time calculation of the sonic intensity estimate in the protected zone for instance when there is external or internal motion of the subject's anatomy.

In another embodiment the selection of the active transducer elements can be handled using the general mathematical formulation known as linear program. The sensitive regions are divided into sufficiently small subregions. One estimates and stores the acoustic intensity exposure caused by each transducer element, driven at unity power, on each sensitive subregion. The estimated total intensity exposure on each subregion can be expressed as a linear equation, where the element powers are the unknowns, and the exposures for unity power appear as coefficients. Requiring that the intensity exposure on each subregion remains below the corresponding safety limits, we have a system of linear inequalities.

In another embodiment the output powers of individual transducer elements remain bound between zero and some maximum value.

In another embodiment the total output power is fixed, we have a well-defined linear program. There are many well-known algorithms for solving linear programs. The individual transducer element powers could also be handled as discrete variables: the elements are either switched off, or have a fixed output power. In this case, the formulation above results in a combinatorial optimization problem.

The 'coherent sum' as used herein encompasses adding all pressure with a specific phase (or as complex number) to get the total pressure field and after that to take square modulus of this the total pressure field to get the intensity pressure (the one really achieved).

However, the phase applied on each transducer element can be changed especially to move electronically the focal point. Thus the maximal intensity coherent sum is calculated by adding the module of the pressure from each element (rather than the pressure as a complex number with a phase) and to take the square of this sum. It corresponds to the maximal intensity distribution achievable when moving the focal point along all direction (or using all possible phase for all channels). This maximal in maximal intensity coherent could be eventually useful to evaluate the difference between the coherent and non coherent sum. It provides the advantage to consider all possible focal point steering.

Alternatively we can consider maximal intensity coherent sum by processing only the maximal intensity distribution achieved when moving the focal point along predefined trajectory such as typical volumetric sonication implemented on the Philips MR-HIFU Sonalleve system.

In another embodiment the protected zone comprises multiple disconnected volumes. This may be beneficial because there may be more than one organ or region of the subject which should be protected from accidental or unintentional sonication. This may have the benefit of reducing the internal damage to a subject.

In another aspect the invention provides for a computer program product comprising machine-executable instructions for execution by a processor controlling a medical instrument. The medical instrument comprises a high-intensity focused ultrasound system comprising an ultrasonic transducer. The ultrasonic transducer comprises multiple transducer elements. The high-intensity focused ultrasound system is operable for switching on and off the supply of electrical power to each of the multiple transducer elements. Execution of the instructions causes the processor to receive a treatment plan specifying a protected zone within a subject. Execution of the instructions further causes the processor to calculate a set of transducer control parameters using the treatment plan. The set of transducer element states specify the switching of electrical power to each of the multiple transducer elements. An ultrasonic intensity estimate may be calculated in this step. The ultrasonic intensity estimate in the protected zone is below a predetermined threshold. The ultrasonic intensity estimate is calculated using an incoherence sum of the ultrasonic pressure generated by each of the multiple transducer elements. The advantages of this have been previously discussed.

In another aspect the invention provides for a method of operating a medical instrument comprising a high-intensity focused ultrasound system which comprises an ultrasonic transducer. The ultrasonic transducer comprises multiple transducer elements. The high-intensity focused ultrasound system is operable for switching on and off the supply of electrical power to each of the multiple transducer elements. The method comprises the step of receiving a treatment plan specifying a protected zone within the subject. The method further comprises the step of calculating a set of transducer control parameters using the treatment plan. The set of transducer element states specify the switching of electrical power to each of the multiple transducer elements. An ultrasonic intensity estimate in the protected zone is below a predetermined threshold. The ultrasonic intensity estimate is calculated using an incoherent sum of the ultrasonic pressure generated by each of the multiple transducer elements. The advantages of this have been previously discussed.

### BRIEF DESCRIPTION OF THE DRAWINGS

In the following preferred embodiments of the invention will be described, by way of example only, and with reference to the drawings in which:
Fig. 1 shows a flow diagram which illustrates as method according to an embodiment of the invention;
Fig. 2 shows a flow diagram which illustrates as method according to a further embodiment of the invention;
Fig. 3 illustrates a medical instrument according to an embodiment of the invention;
Fig. 4 shows a detailed drawing showing of an ultrasound transducer and a subject;
Fig. 5 illustrates a medical instrument according to a further embodiment of the invention;
Fig. 6 illustrates a medical instrument according to a further embodiment of the invention;
Fig. 7 shows the simulated logarithmic acoustic intensity from a single transducer element in a water tank;
Fig. 8 shows the simulated logarithmic acoustic intensity from multiple transducer elements in a water tank;
Fig. 9 shows an incoherent sum of element powers from individual transducer elements for the same calculation as was shown in Fig. 8; and
Fig. 10 shows the ratio of the logarithmic acoustic intensity and the incoherent power sum from Figs. 8 and 9.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

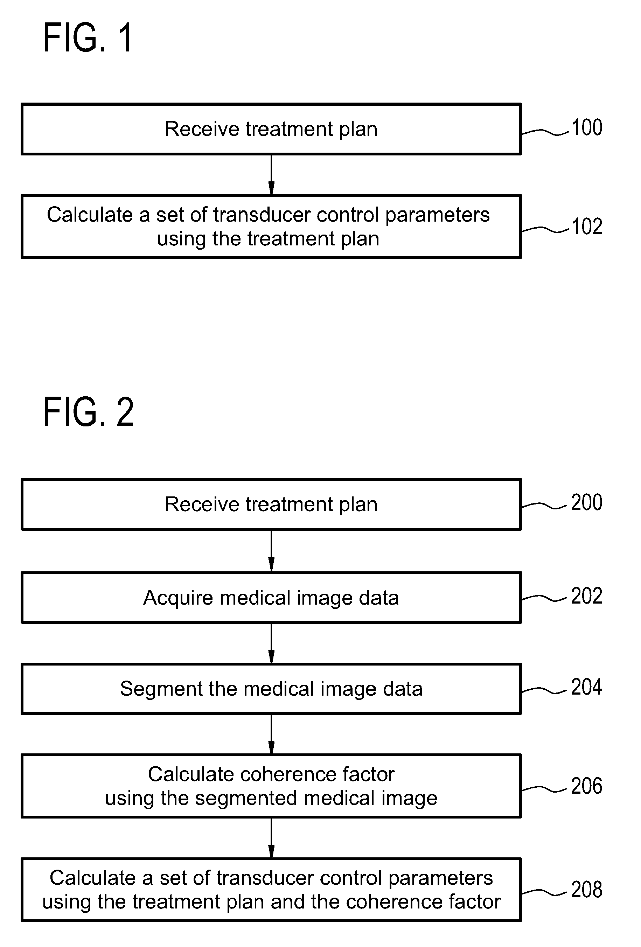

Fig. 1 shows a flow diagram which illustrates as method according to an embodiment of the invention. In step 100 a treatment plan is received. The treatment plan specifies a protected zone within a subject. Finally in step 102 a set of transducer control parameters are calculated using the treatment plan. The set of transducer control parameters specify the switching of electrical power to each of the multiple transducer elements. An ultrasonic intensity estimate may be calculated in this step. The ultrasonic intensity estimate in the protected zone is below a predetermined threshold. The ultrasonic intensity estimate is calculated using an incoherent sum of the ultrasonic pressure generated by each of the multiple transducer elements.

Fig. 2 shows a flow diagram which illustrates a further embodiment of the invention. In step 200 a treatment plan specifying a protected zone within a subject is received. Next in step 202 medical image data is acquired using a medical imaging system. Next in step 204 the medical image data is segmented to identify different tissue types or regions within the subject. Next in step 206 a coherence factor is calculated using the segmented medical image and a model of the transducer. Then finally in step 208 a set of transducer control parameters is calculated using the treatment plan and the coherence factor. In particular the coherence factor in this embodiment may be spatially-dependent.

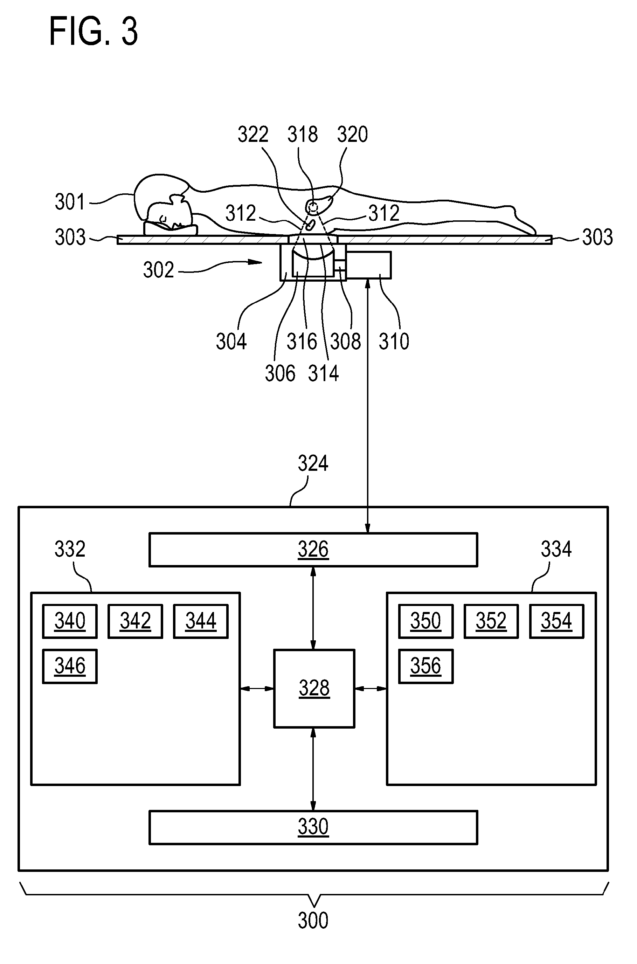

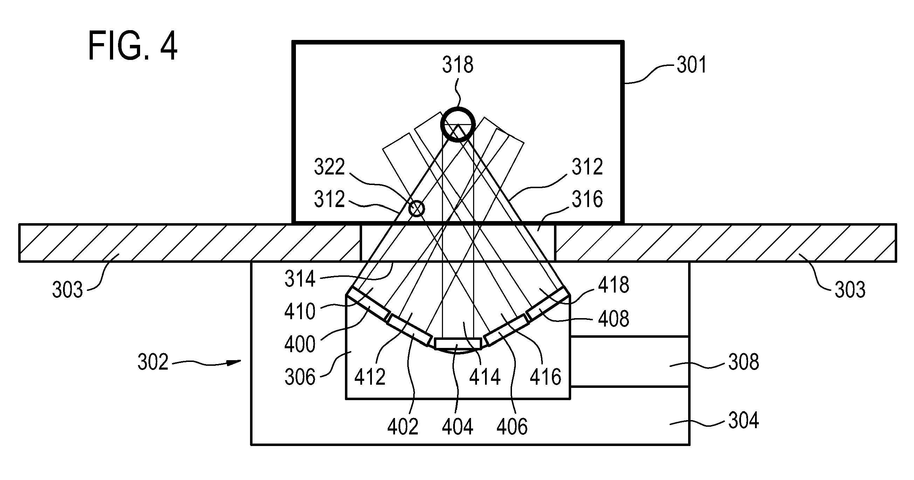

Fig. 3 illustrates a medical instrument 300 according to an embodiment of the invention. In addition to the components, the embodiment shown in Fig. 4 comprises a temperature treatment system which is a high-intensity focused ultrasound system 302 for sonicating a subject 301. The high-intensity focused ultrasound system is mounted below a subject support 303. The subject 301 is resting on the subject support 303. The high-intensity focused ultrasound system comprises a fluid-filled chamber 304. Within the fluid-filled chamber 304 is an ultrasound transducer 306. Although it is not shown in this figure the ultrasound transducer 306 may comprise multiple ultrasound transducer elements each capable of generating an individual beam of ultrasound. This may be used to steer the location of a sonication point 318 electronically by controlling the phase and/or amplitude of alternating electrical current supplied to each of the ultrasound transducer elements.

The ultrasound transducer 306 is connected to a mechanism 308 which allows the ultrasound transducer 306 to be repositioned mechanically. The mechanism 308 is connected to a mechanical actuator 310 which is adapted for actuating the mechanism 308. The mechanical actuator 310 also represents a power supply for supplying electrical power to the ultrasound transducer 306. In some embodiments the power supply may control the phase and/or amplitude of electrical power to individual ultrasound transducer elements. The ultrasound transducer 306 generates ultrasound which is shown as following the path 312. The ultrasound 312 goes through the fluid-filled chamber 308 and through an ultrasound window 314. In this embodiment the ultrasound then passes through a gel pad 316. The gel pad is not necessarily present in all embodiments but in this embodiment there is a recess in the subject support 303 for receiving a gel pad 316. The gel pad 316 helps couple ultrasonic power between the transducer 306 and the subject 301. After passing through the gel pad 316 the ultrasound 312 passes through the subject 301 and is focused to a sonication point 318. The sonication point is understood to be a finite volume or localized volume to which the ultrasound is focused. The sonication point 318 is being focused within a target zone 320.

The sonication point 318 can be seen as being located within target zone 320. The target zone 320 may be a collection of sonication points to be sonicated at sequential time intervals. Between the sonication point 318 and the ultrasonic transducer 306 is a protected zone 322. This is a portion of the subject 301 which is desired to be protected from being sonicated or damaged by the ultrasound.

The high intensity focused ultrasound system 302 is shown as being connected to a hardware interface 326 of the computer 324. The hardware interface 326 is connected to a processor 328. The hardware interface 326 enables the processor 328 to send and receive data and commands to control the operation and function of the medical instrument 300. The processor 328 is further connected to a user interface 330, computer storage 332 and computer memory 334.

The computer storage 332 is shown as containing a treatment plan 340. The computer storage 332 is further shown as containing a set of transducer control parameters 342. The transducer control parameters 342 contain at the least a specification of which of the multiple transducer elements to turn on and off during sonication of the target zone 320. Depending upon the sonication point, the specification of which transducers to turn on and off may be different. The computer storage 332 is further shown as containing sonication control commands 344. The sonication control commands 344 are commands which cause the high-intensity focused ultrasound system 302 to sonicate the target zone 320.

The computer memory 334 is shown as containing a control module 350. The control module 350 contains computer-executable code which enables the processor 328 to control the operation and function of the medical instrument 300. The computer memory 334 is further shown as containing a transducer control parameter generation module 352. The transducer control parameter generation module 352 contains computer-executable instructions which enable the processor 328 to calculate the transducer control parameters using at least the treatment plan 340. In other embodiments medical image data and/or a coherence factor may also be used to calculate the transducer control parameters.

The computer storage 332 is further shown as containing a predetermined threshold 346 of the maximum ultrasonic intensity allowed within the protected zone 322. In some embodiments the predetermined threshold 346 is spatially-dependent.

The computer memory 334 is further shown as containing a sonication control command generation module 354. The sonication control command generation module 354 comprises computer-executable instructions which enable the processor 328 to calculate the sonication control commands 344 using at least the transducer control parameters 342 and possibly the treatment plan 340.

The computer memory may also contain an ultrasonic modeling module 356. The ultrasonic modeling module contains computer executable instructions for modeling the ultrasonic transducer 306. The control parameter generation module 352 and/or the sonication control command generation module 354 may use the ultrasonic modeling module. In some embodiments the ultrasonic modeling module 356 may model such things as the transducer 306 geometry, the location of each transducer element, the shape of the transducer elements (i.e., the diameter for ciruclar element), and the focal length of the transducer and the operating frequency.

In some embodiments the ultrasonic modeling module may be used for modeling an electronically adjustable focus for focusing ultrasonic energy on into a target zone and the control parameters necessary for adjusting the focus. For example in some embodimetns the module may be used for calculating a set of time dependent controlling phases such that electronically adjustable focus follows a path. This may then be used to calculate the coherent sum at least partially using the set of time dependent controlling phases.

In some embodiments the ultrasonic modeling module 356, the control parameter generation module 352, and/or the sonication control command generation module 354 may be used to determine additional parameters such as: the phase of electrical power supplied to each of the multiple transducer elements, the amplitude of electrical power supplied to each of the multiple transducer elements, the power level of the electrical power supplied to each of the multiple transducer elements, the alternating frequency of electrical power to each of the multiple transducer elements, the duration of electrical power supplied to each of the multiple transducer elements, and/or the sonication trajectory of the focus of the ultrasonic transducer.

Fig. 4 shows a more detailed drawing showing the ultrasound transducer 306 and the subject 301. In this Fig. the ultrasonic transducer 306 is shown with five ultrasonic transducer elements 400, 402, 404, 406, 408. A first transducer element 400 is shown, a second transducer element 402 is shown, a third transducer element 404 is shown, a fourth transducer element 406 is shown and a fifth transducer element 408 is shown. It should be noted that this Fig. is highly idealized and normally several hundred transducer elements may be present on the surface of the ultrasonic transducer 306.

Rectangles 410, 412, 414, 416, 418 abutting the transducer elements 400, 402, 404, 406, 408 are idealized to represent ultrasonic energy coming from each of the transducer elements 400, 402, 404, 406, 408. The purpose of these rectangles is to illustrate where a bulk of the ultrasound generated by a particular transducer element 400, 402, 404, 406, 408 may travel. Rectangle 410 is the ultrasound from transducer element 1 400. Rectangle 412 is the ultrasound from transducer element two 402. Rectangle 414 represents the ultrasound from transducer element three 404. Rectangle 416 represents the ultrasound from transducer element four 406. Rectangle 418 represents the ultrasound from transducer element five 408. Examining the Fig. it can be seen that rectangles 410 and 416 come in contact with the protected zone 322. In this idealized situation the electrical power supplied to transducer element one (400) and transducer element four 406 may be switched off to reduce the likelihood of heating the protected zone 322.

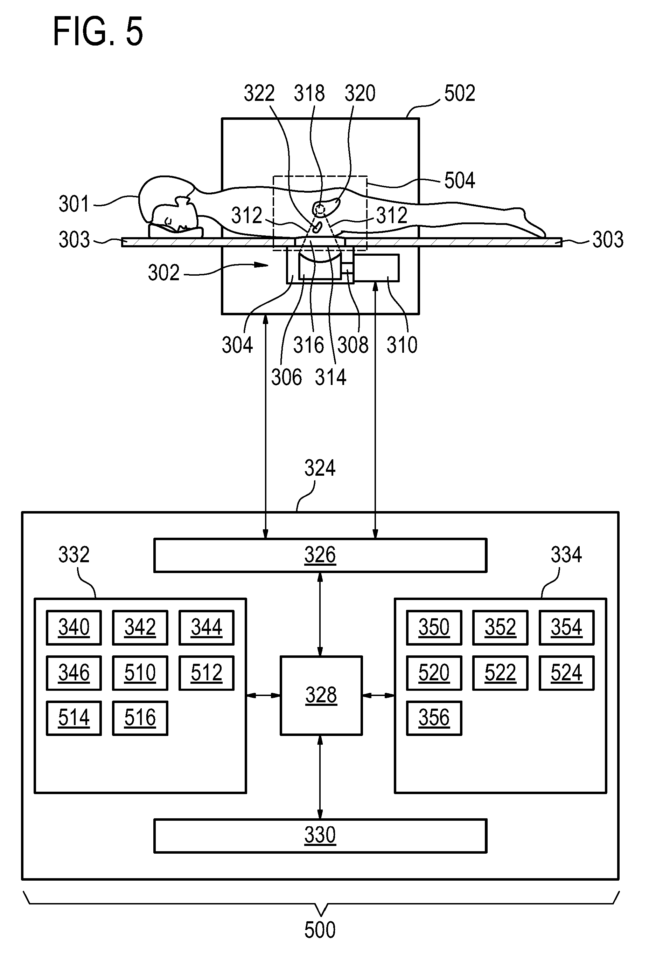

Fig. 5 shows a medical instrument 500 according to a further embodiment of the invention. The embodiment shown in Fig. 5 is similar to the embodiment shown in Fig. 3 except in this embodiment there is the addition of a medical imaging system 502. The medical imaging system may represent a variety of medical imaging systems. Since the medical imaging system may be, but is not limited to: a computer tomography system, a magnetic resonance imaging system, and a diagnostic ultrasound system. In this idealized medical imaging system 502 there is an imaging zone 504 where medical image data 510 may be acquired from. The computer storage 332 shows the medical image data 510 that is acquired using the medical imaging system 502. The computer storage 332 is further shown as containing a medical image 512 that has been reconstructed from the medical image data 510. The computer storage 332 is further shown as containing an image segmentation 514 which has been calculated from the medical image 512. The image segmentation 514 may be used to identify different tissue types within the subject 301 and also to register the treatment plan 340 to the anatomy of the subject 301. The computer storage 332 further shows a coherence factor 516 that has been calculated using the image segmentation 514.

The computer memory 334 is further shown as containing an image reconstruction module 520. The image reconstruction module 520 contains computer-executable instructions which enable the processor 328 to reconstruct the medical image 512 from the medical image data 510. The computer memory 334 is further shown as containing an image segmentation module 522 which comprises computer-executable code which enables the processor 328 to create the image segmentation 514 from the medical image 512. The computer memory 334 is shown as further containing a coherence factor calculation module 524. The coherence factor calculation module 524 may be used to create a spatially dependent coherence factor 516 from the image segmentation 514.

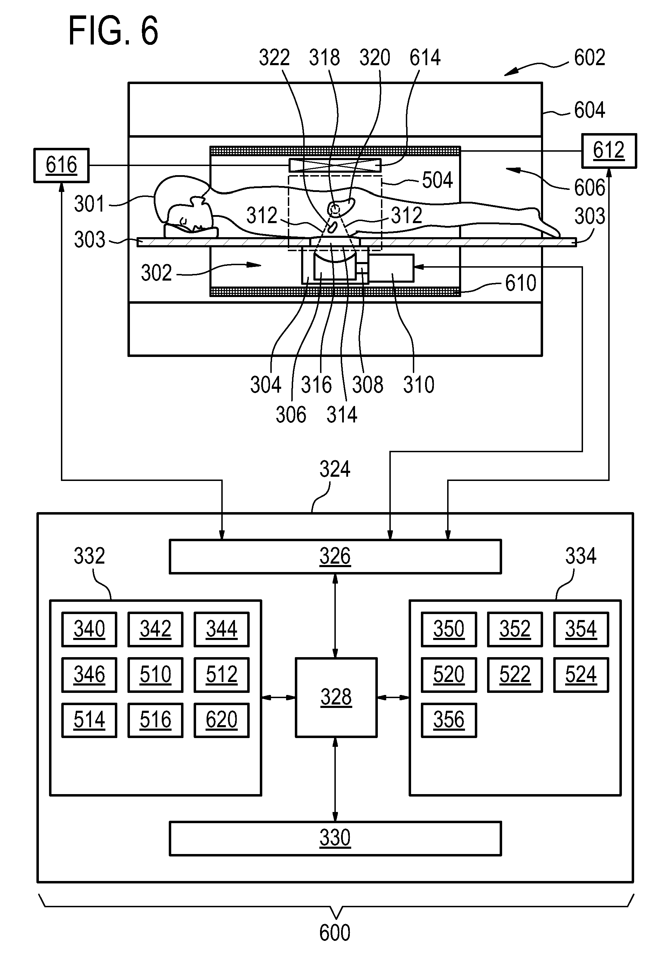

Fig. 6 shows a medical instrument 600 according to a further embodiment of the invention. The medical instrument 600 shown in Fig. 6 is similar to the medical instrument 300 shown in Fig. 3. The medical instrument 600 comprises a magnetic resonance imaging system 602. The magnetic resonance imaging system comprises a magnet 604. The magnet 604 is a cylindrical type superconducting magnet with a bore 606 through the center of it. The magnet has a liquid helium cooled cryostat with superconducting coils. It is also possible to use permanent or resistive magnets. The use of different types of magnets is also possible for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 606 of the cylindrical magnet there is an imaging zone 504 where the magnetic field is strong and uniform enough to perform magnetic resonance imaging.

Within the bore 606 of the magnet there is also a set of magnetic field gradient coils 610 which are used for acquisition of magnetic resonance data to spatially encode magnetic spins within the imaging zone 504 of the magnet 604. The magnetic field gradient coils are connected to a magnetic field gradient coil power supply 612. The magnetic field gradient coils 610 are intended to be representative. Typically magnetic field gradient coils contain three separate sets of coils for spatially encoding in three orthogonal spatial directions. A magnetic field gradient power supply 612 supplies current to the magnetic field gradient coils 610. The current supplied to the magnetic field coils is controlled as a function of time and may be ramped or pulsed.

Adjacent to the imaging zone 504 is a radio-frequency coil 614 for manipulating the orientations of magnetic spins within the imaging zone 504 and for receiving radio transmissions from spins also within the imaging zone. The radio-frequency coil may contain multiple coil elements. The radio-frequency coil may also be referred to as a channel or an antenna. The radio-frequency coil 614 is connected to a radio frequency transceiver 616. The radio-frequency coil 614 and radio frequency transceiver 616 may be replaced by separate transmit and receive coils and a separate transmitter and receiver. It is understood that the radio-frequency coil 614 and the radio-frequency transceiver 616 are representative. The radio-frequency coil 614 is intended to also represent a dedicated transmit antenna and a dedicated receive antenna. Likewise the transceiver 616 may also represent a separate transmitter and receivers.

The computer storage 332 is shown as additionally containing a pulse sequence 620. A pulse sequence as used herein encompasses a set of instructions which enables the processor 328 to control the magnetic resonance imaging system 402 to acquire the medical image data 510, which in this embodiment is magnetic resonance data.

In High Intensity Focused Ultrasound (HIFU) treatment, a focused ultrasound beam is used to selectively heat tissue inside the patient. During the treatment, it may be beneficial if care is taken to avoid undesired heating of sensitive organs, such as scars, bowels, or bones located on the beam path. It has been demonstrated that undesired ultrasound exposure can be reduced by selectively turning off transducer elements. This invention describes an algorithm for choosing the active elements in a tightly controlled way, without compromises on the safety of the patient. According to the invention, the sensitive regions are identified and marked, and a safety level is associated with each sensitive region. The intensity exposure on each sensitive region is estimated based on the incoherent and maximally coherent sum of the estimated intensities from transducer elements. Elements are turned off until the estimated ultrasound exposure is below safety level on all sensitive regions.

As mentioned above, a focused ultrasound beam is used in HIFU treatment to selectively heat tissue inside the patient. A focusing ultrasound transducer is used to generate the beam. The shape of the beam resembles a cone, with the transducer forming the base of the cone. The beam converges towards a focal point, which forms the apex of the cone. At the focal point, the pressure waves generated at the transducer sum up coherently, resulting in a sharp spot where the acoustic intensity is very high. Because absorption is proportional to the intensity, highly localized heating takes place. For steerability, the transducer surface may be divided into independent transducer elements. The location of the focal point can be shifted by adjusting the phases of the transducer elements electrically.

Also the tissue located on the beam path gets heated. However, the incoming ultrasound beam is distributed over a large area of skin. Moreover, far from the focus the beams from individual transducer elements are incoherent. Consequently, normally the undesired heating remains limited and is clinically harmless. However, certain anatomic regions, such as scars, bowels, and bones, are highly sensitive to ultrasound. Attention may be paid on avoiding the exposure of these regions to ultrasound. Presently, this is done manually in the treatment planning software. The sensitive regions are identified from MRI images, and the transducer is manually positioned in such a way that the ultrasound beam, visualized as a cone, does not hit the sensitive regions. In the uterine fibroid treatment, this can significantly limit the accessible treatment volume. In some potential future applications, such as liver cancer treatment, this approach is extremely limited due to the presence of ribs.

Suppressing the acoustic intensity locally by selectively turning off transducer elements has received much interest lately. In algorithms based on a "geometric" approach, a part of the transducer surface is considered to be in the shadow of the sensitive regions, as seen from the focal point. The transducer elements in the shadow are turned off. In algorithms based on the "diffraction" approach, the focus is treated as a source. The propagation of an acoustic field from the focus towards the transducer is modeled. The sensitive regions can be applied as a spatial mask, which removes part of the acoustic field. The wave field arriving at the transducer is used to select the active transducer elements and the phases of the active elements.

While the specified sensitive regions (typically, ribs) get mapped into a configuration of active elements, and reduction of acoustic exposure on the sensitive regions can be expected, the reduction is not quantified. The exposure could be estimated afterwards, but it is not utilized in the element selection.

In an embodiment of the invention, quantitative safety limits are specified for each sensitive region. The quantitative limits can be based on qualitative classification by the user and the planned sonication. The algorithm estimates the exposure on each region, and attempts to find such a configuration of active elements that the safety limits are obeyed. If such a solution is not found, the sonication is considered unfeasible.

The proposed algorithm may have other advantages as well. For example, the ultrasonic beam is affected by the properties of tissues and materials on the acoustic path. In particular, refraction at material interfaces and acoustic attenuation in tissues are important for the magnitude and spatial distribution of the acoustic intensity. It is difficult to include such factors in the geometric approach. The proposed algorithm can be made to utilize segmented patient models and a suitable simulation engine, should those be available.

Current approaches model the overall acoustic field, without considering the beams from individual elements. Consequently, the acoustic field in the near-field is not properly described. In extreme cases, even the side lobes might have consequences on the patient safety. Such extreme cases are probably unfeasible for treatment, but for the sake of the patient safety it is important to recognize them. A proper description of the near-field structure is inherent for the proposed algorithm.

All the topics mentioned here could be solved also by an algorithm utilizing full-wave simulations, preferably with associated full segmented patient model. However, the computational (and other) demands set by such an algorithm may be excessive, making the approach unfeasible for clinical application. The computational demands of an embodiment of the invention may be modest and adjustable.

In another embodiment reflections from mechanical structures located on the beam path are minimized and/or reduced. For example, in some cases it may necessary to position the transducer in such way that the mechanical support structures partly overlap the acoustic window. Upon hitting such structures, part of the acoustic beam may be reflected. In unfortunate conditions the reflected beam may become focused on the transducer surface, heating the transducer, and possibly breaking elements. Suppressing the acoustic intensity incident on such structures reduces this risk.

An embodiment of the invention may be an algorithm for selecting which transducer elements are turned off and which are turned on. Clinically, the key improvement as compared to prior art is that the algorithm is quantitative by nature: safety limits can be specified and enforced on the sensitive regions. From technical perspective, the discovery which makes the invention possible is a new method to compute upper-limit estimates on the acoustic intensity.

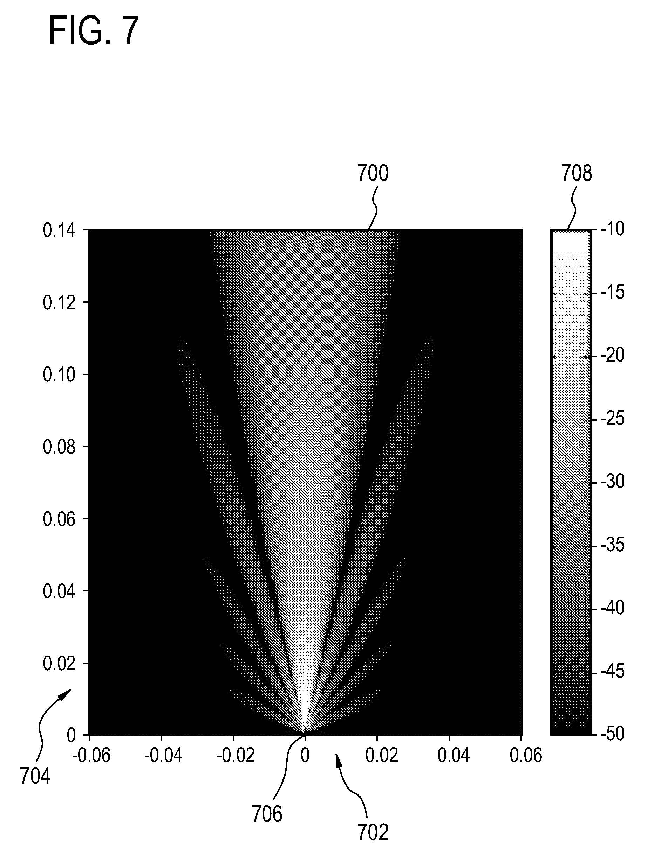

An embodiment of the method may be understood by examining Figs. 7 through 10. In the context of interest here, the beam from an ultrasonic transducer consists of the beams emitted by individual transducer elements.

Fig. 7 shows the logarithmic acoustic intensity from a single transducer element in a water tank. The axial plane is shown. The image 700 shows the logarithmic acoustic intensity. The x-axis is labeled 702 and is in units of meters. The y-axis is labeled 704 and is also in units of meters. The point 706 is the location of the single transducer element. The change in intensity is shown on the scale 708 and is measured in the change intensity in decibels. The intensity is measured relatively in watts per square centimeter. Fig. 7 shows the acoustic intensity from a single transducer element, simulated in axial plane. For simplicity of the demonstration, a water tank is considered as the medium.

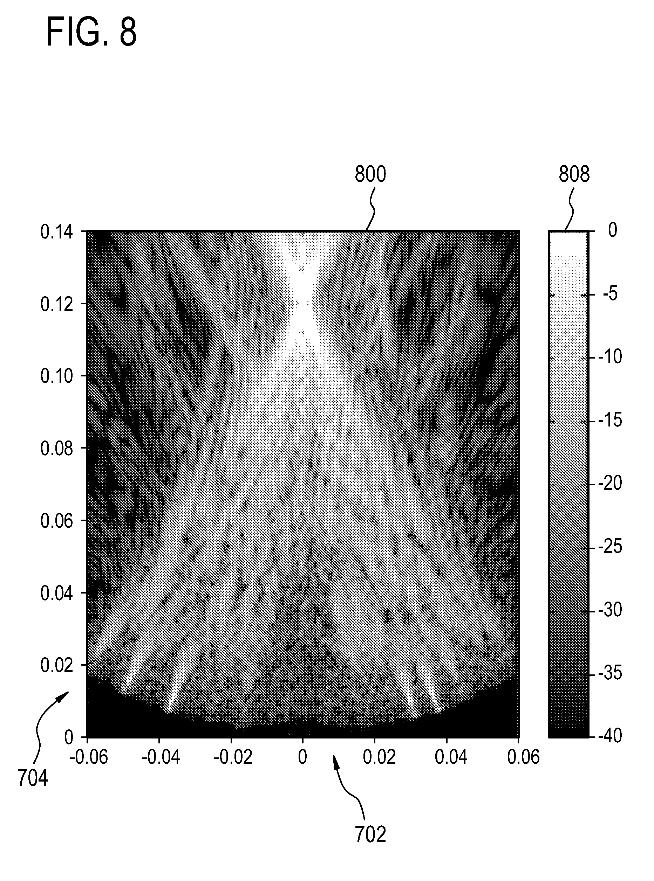

Fig. 8 shows the logarithmic acoustic intensity from multiple transducer elements in a water tank also on the axial plane. The image 800 shows the logarithmic acoustic intensity in the axial plane and the scale 808 shows the change in the intensity in decibels. The intensity change is relative and is in watts per square centimeter. Fig. 8 shows the simulated intensity from a transducer consisting of 256 transducer elements, under the same conditions. The focus of the transducer is located at 120 mm depth into the tank. At the focus, the beams from individual elements add up coherently, resulting in 256-fold increase in pressure, and 256² = 65536-fold increase [48 dB] in the acoustic intensity, as compared to a single element.

By turning off transducer elements one seeks to protect sensitive organs located between the transducer and the focus. One can observe that in this region the spatial structure of the ultrasound field is very complicated. This is due to random-like interference between the elements. Applying electric deflection further changes the structure of the field. A typical sonication involves several dozen different electric deflections. Consequently, estimating the peak intensity over an extended 3D volume by exhaustive search becomes a very laborious task.

Fortunately, for safety considerations it is not necessary to know the detailed structure: it suffices to know the local level of the hot spots. This can be expected to be a much more smooth quantity. As the ultimate worst-case estimate, one can assume that the elements are always coherent, and sum the magnitudes of single-element pressure fields. In most cases, this approach massively overestimates the intensity levels.

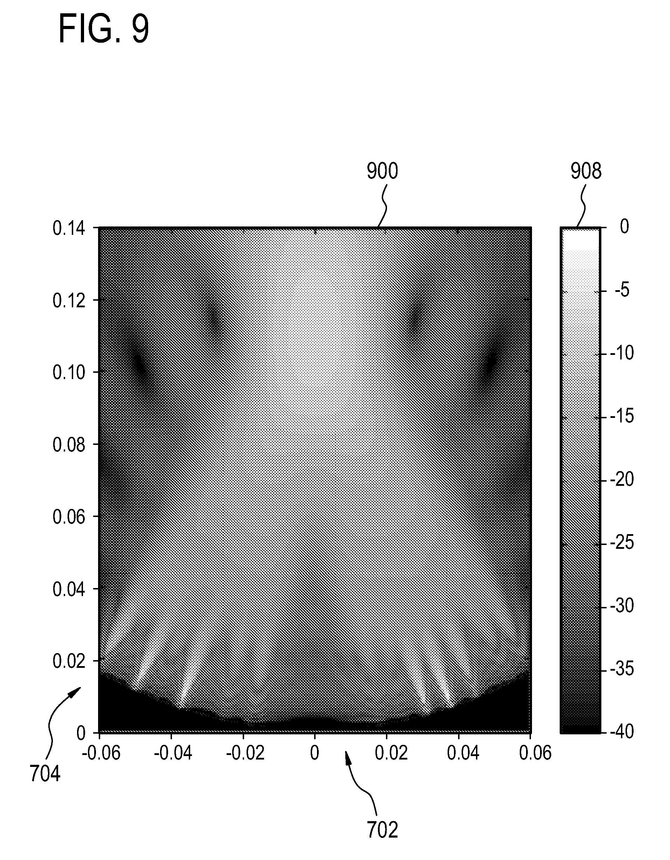

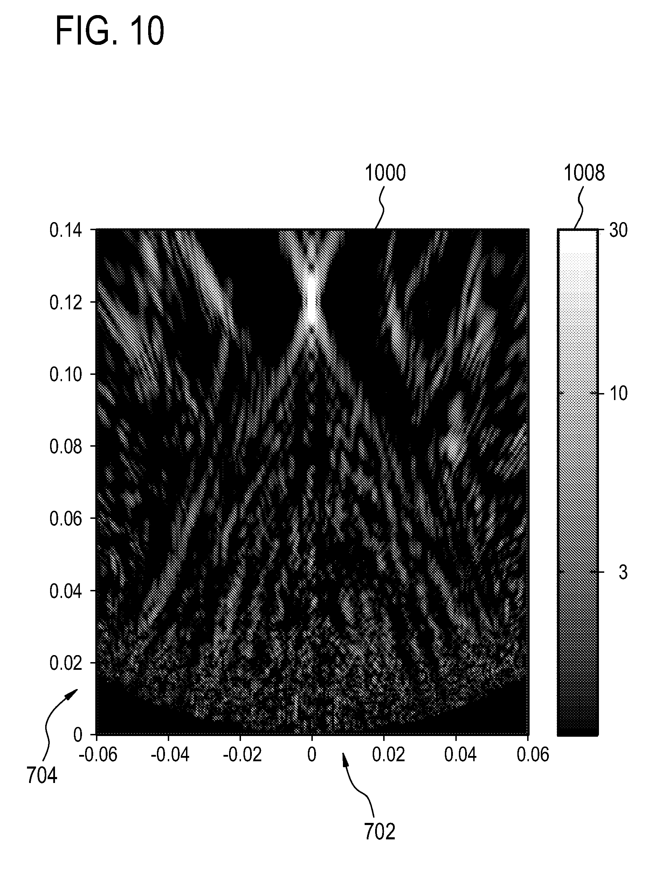

As the opposite case, one can assume that the elements are incoherent, and sum up the single-element intensities. This approach underestimates the hot spots. However, practical examination shows that the approach actually works quite well. The incoherent sum for the water tank example is shown in Fig. 9 and the ratio of the intensity by the incoherent sum is shown in Fig. 10. Excluding the focus region, the ratio remains below 8 or so. Hence, an upper estimate on the intensity can be obtained summing up the individual element intensities and multiplying by a constant coefficient (in this case, say, 10).

Fig. 9 shows an incoherent sum of element powers from individual transducer elements for the same calculation as was shown in Fig. 8. The image 900 shows the logarithmic incoherent power sum 900 the change in intensity is shown on scale 908 in decibels. The power was calculated in terms of watts per square centimeter. In comparing Figs. 8 and 9 it can be seen that overall the hot regions in Fig. 9 encompass the hot regions shown in Fig. 8. This shows how the logarithmic incoherent power sum 900 particularly when multiplied by a coherence factor may be used to approximate the logarithmic acoustic intensity 800.

Fig. 10 shows the ratio of the logarithmic acoustic intensity and the incoherent power sum 900. The image 1000 shows this ratio and the scale is shown as 1008. Essentially Fig. 10 could be used for calculating a coherence factor or may be useful for illustrating the use of the incoherent power sum 900 instead of the acoustic intensity 800. In the example illustrated in Figs. 8, 9 and 10 a constant value for the coherence factor of 8 would be sufficient to enable the safe use of the incoherent sum for calculating the ultrasonic intensity estimate in the protected zone.

For more complicated geometries, involving a segmented model of the patient and perhaps a more difficult transducer, the approach can be refined a bit. The single-element intensity maps can be evaluated based on the segmented anatomic data. The intensity estimate can be obtained by weighting the incoherent and maximally coherent sums. The weighting can have spatial dependence.

The sensitive regions can be identified and segmented as a part of treatment planning. A simple approach is that the user marks them manually on treatment planning console, based on the planning MRI images. Alternatively, automatic or semiautomatic segmentation could be used. The sensitive regions could also be segmented prior to the treatment session, and some sort of image registration could be used to update the segmented models in the beginning of the treatment.

Safety levels can be specified on the sensitive regions. For example, user could classify the sensitive regions based on the tissue type, or classify the regions into few safety categories. Software could determine the safety limits accordingly. Mostly likely the safety levels should depend on the length of the sonication and the tissue/organ in question.

The segmented sensitive regions may to be divided into sufficiently small partial volumes. If the algorithm is implemented correctly, using too coarse division should result in the algorithm shutting off more elements than necessary. Means can be provided to form an upper estimate on the acoustic intensity from each element on each sensitive sub-volume. In absence of better knowledge, a simple approach is to treat the propagation path as homogeneous medium. If segmented patient model is available, it would be beneficial to use it in the estimation. The element-specific estimates are stored.

A coherence factor may be defined. A simple approach is to use a transducer-specific constant. In the example above, say, value 10 could be used as the margin. For some transducers it might be advantageous to define a value depending on the position relative to the transducer.

In an embodiment a configuration of active elements that the safety levels are obeyed on all sensitive regions is found. It is easy to compose alternative strategies for achieving this goal. For example, the following heuristic algorithm could be applied.

In the beginning, one may assume that all elements are active. Power level is specified and divided amongst the elements. One goes through all sensitive regions, and forms an upper estimate on the acoustic exposure. The estimate is computed as the incoherent sum of the element intensities, multiplied by the coherence factor. If the estimated intensity exceeds the safety level on any of the sensitive regions, elements are shut off. For example, one could pick up the sub-volume where the highest violation of the safety level takes place, and shut off the element giving the highest contribution there. The power of the remaining active elements is increased accordingly. The process is iterated until the estimated intensity is below the safety criteria on all sensitive regions, or until the sonication is identified as unfeasible.

In another embodiment, one defines two coherence factors: one for the incoherent sum, another one for the maximally coherent sum, and forms the intensity estimate from these. Either or both of the factors can have a spatial dependence.

For the purpose of avoiding reflections from mechanical structures, the decision to turn off an element can also be based on the angle between the beam on the structure.

In another embodiment, the feasibility of the new active element configuration can be checked, using a defined set of rules. For example, one could require that there is at least a certain number of active elements.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 300: medical instrument
- 301: subject
- 302: high intensity focused ultrasound system
- 303: subject support
- 304: fluid filled chamber
- 306: ultrasound transducer
- 308: mechanism
- 310: mechanical actuator/power supply
- 312: path of ultrasound
- 314: ultrasound window
- 316: gel pad
- 318: sonication point
- 320: target zone
- 322: protected zone
- 324: computer
- 326: hardware interface
- 328: processor
- 330: user interface
- 332: computer storage
- 334: computer memory
- 340: treatment plan
- 342: transducer control parameters
- 344: sonication control commands
- 346: predetermined threshold
- 350: control module
- 352: transducer control parameter generation module
- 354: sonication control command generation module
- 356: ultrasonic modeling module
- 400: transducer element one
- 402: transducer element two
- 404: transducer element three
- 406: transducer element four
- 408: transducer element five
- 410: ultrasound from transducer element one
- 412: ultrasound from transducer element two
- 414: ultrasound from transducer element three
- 416: ultrasound from transducer element four
- 418: ultrasound from transducer element five
- 500: medical instrument
- 502: medical imaging system
- 504: imaging zone
- 510: medical image data
- 512: medical image
- 514: image segmentation
- 516: coherence factor
- 520: image reconstruction module
- 522: image segmentation module
- 524: coherence factor calculation module
- 600: medical instrument
- 602: magnetic resonance imaging system
- 604: magnet
- 606: bore of magnet
- 610: magnetic field gradient coils
- 612: magnetic field gradient coils power supply
- 614: radio-frequency coil
- 616: transceiver
- 620: pulse sequence
- 700: logarithmic acoustic intensity
- 702: x-direction [m]
- 704: y-direction [m]
- 706: location of transducer
- 708: change in intensity [dB]
- 800: logarithmic acoustic intensity
- 808: change in intensity [dB]
- 900: logarithmic incoherent power sum
- 908: change in intensity [dB]
- 1000: logarithmic ratio of intensity and incoherent power sum
- 1008: change in intensity [dB]

## Claims

1. A medical instrument (300, 500, 600) comprising:
- a high intensity focused ultrasound system (302) comprising an ultrasonic transducer (306); wherein the ultrasonic transducer comprises multiple transducer elements (400, 402, 404, 406, 408), wherein the high intensity focused ultrasound system is operable for switching on and off the supply of electrical power to each of the multiple transducer elements ;
- a processor (328) for controlling the medical instrument;
- a memory (334) containing machine executable instructions (350, 352, 354, 520, 522, 524); wherein execution of the instructions causes the processor to:
- receive (100, 200) a treatment plan (340) specifying a protected zone (322) within a subject (301);
- calculate (102, 208) a set of transducer control parameters (342) using the treatment plan such that an ultrasonic intensity estimate (900) in the protected zone is below a predetermined threshold, wherein the set of transducer control parameters specify the switching of electrical power to each of the multiple transducer elements, **characterized in that**
- the ultrasonic intensity estimate is calculated using an incoherent sum of the ultrasonic pressure generated by each of the multiple transducer elements.

2. The medical instrument of claim 1, wherein the incoherent sum of the ultrasonic pressure generated by each of the multiple transducer elements is multiplied by a coherence factor (516, 1000) to calculate the ultrasonic intensity estimate.

3. The medical instrument of claim 2, wherein the coherence factor is spatially dependent.

4. The medical instrument of claim 3, wherein the medical instrument further comprises a medical imaging system (502, 602) for acquiring medical image data (510) within an imaging zone (504), wherein execution of the instructions further causes the processor to acquire (202) the medical image data, wherein the protected zone is within the imaging zone, and wherein the set of transducer control parameters are calculated at least partially using the medical image data.

5. The medical instrument of claim 4, wherein execution of the instructions further causes the processor to:
- calculate (204) an image segmentation (514) using the medical image data, wherein the image segmentation identifies tissue types within the subject; and
- calculate (206) the coherence factor at least partially using the image segmentation.

6. The medical instrument of claim 4 or 5, wherein the medical imaging system is any one of the following: a computed tomography system, a magnetic resonance imaging system (602), and a diagnostic ultrasound system.

7. The medical instrument of any one of claims 3 through 6, wherein the coherence factor is calculated at least partially using a coherent sum (800) of the ultrasonic pressure generated by each of the multiple transducer elements.

8. The medical instrument of claim 7, wherein the ultrasonic transducer has an electronically adjustable focus for focusing ultrasonic energy on into a target zone, wherein the high intensity focused ultrasound system is operable for controlling the electronically adjustable focus by controlling the phase of electrical power to each of the multiple transducer elements, wherein the target zone is a path, wherein execution of the instructions further causes the processor to:
- calculate a set of time dependent controlling phases, wherein the time dependent controlling phases specify the phase of electrical power supplied to each of the multiple transducer elements as a function of time such that the electronically adjustable focus follows the path; and
- calculate the coherent sum at least partially using the set of time dependent controlling phases.

9. The medical instrument of any one of the preceding claims, wherein the set of transducer control parameters further comprise any one of the following: phase of electrical power supplied to each of the multiple transducer elements, amplitude of electrical power supplied to each of the multiple transducer elements, power level of the electrical power supplied to each of the multiple transducer elements, alternating frequency of electrical power to each of the multiple transducer elements, duration of electrical power supplied to each of the multiple transducer elements, sonication trajectory, and combinations thereof

10. The medical instrument of any one of the preceding claims, wherein the set of transducer element parameters is calculated by simulating the switching on and off of combinations of the multiple transducer elements.

11. The medical instrument of any one of the preceding claims, wherein the set of phases is calculated by solving a combinatorial optimization problem.

12. The medical instrument of any one of the preceding claims, wherein execution of the instructions further causes the processor to model at least the protected zone as multiple regions, and wherein the set of transducer element states is solved using a linear programming problem for the multiple regions.

13. The medical instrument of any one of the preceding claims, wherein the protected zone comprises multiple disconnected volumes.

14. A computer program product comprising machine executable instructions (350, 352, 354, 520, 522, 524) for execution by a processor (328) controlling a medical instrument (300, 500, 600), wherein the medical instrument comprises a high intensity focused ultrasound system (302) comprising an ultrasonic transducer (306), wherein the ultrasonic transducer comprises multiple transducer elements (400, 402, 404, 406, 408), wherein the high intensity focused ultrasound system is operable for switching on and off the supply of electrical power to each of the multiple transducer elements, wherein execution of the instructions causes the processor to:
- receive (100, 200) a treatment plan (240) specifying a protected zone (322) within a subject (301);
- calculate (102, 208) a set of transducer control parameters (342) using the treatment plan such that an ultrasonic intensity estimate (900) in the protected zone is below a predetermined threshold, wherein the set of transducer element states specify the switching of electrical power to each of the multiple transducer elements, wherein the ultrasonic intensity estimate is calculated using an incoherent sum of the ultrasonic pressure generated by each of the multiple transducer elements.

15. A method of operating a medical instrument (300, 500, 600) comprising a high intensity focused ultrasound system (302), wherein the high intensity focused ultrasound system comprises an ultrasonic transducer (306), wherein the ultrasonic transducer comprises multiple transducer elements (400, 402, 404, 406, 408), wherein the high intensity focused ultrasound system is operable for switching on and off the supply of electrical power to each of the multiple transducer elements, wherein the method comprises the steps of
- receiving (100, 200) a treatment plan (340) specifying a protected zone (322) within a subject (301);
- calculating (102, 208) a set of transducer control parameters (342) using the treatment plan such that an ultrasonic intensity estimate (900) in the protected zone is below a predetermined threshold, wherein the set of transducer element states specify the switching of electrical power to each of the multiple transducer elements, wherein the ultrasonic intensity estimate is calculated using an incoherent sum of the ultrasonic pressure generated by each of the multiple transducer elements.

## Patentansprüche

1. Medizinisches Instrument (300, 500, 600), das Folgendes umfasst:
- ein mit hochintensivem fokussiertem Ultraschall (engl. high intensity focused ultrasound, HIFU) arbeitendes System (302), das einen Ultraschallwandler (306) umfasst; wobei der Ultraschallwandler eine Vielzahl von Wandlerelementen (400, 402, 404, 406, 408) umfasst, wobei das mit hochintensivem fokussiertem Ultraschall arbeitende System betriebsfähig ist, um die Zuführung von elektrischer Energie zu jedem der Vielzahl von Wandlerelementen ein- und auszuschalten;
- einen Prozessor (328) zum Steuern des medizinischen Instruments;
- einen Speicher (334) mit maschinenausführbaren Anweisungen (350, 352, 354, 520, 522, 524); wobei die Ausführung der Anweisungen den Prozessor veranlasst,
- einen Behandlungsplan (340), der eine geschützte Zone (322) innerhalb eines Patienten (301) spezifiziert, zu empfangen (100, 200);
- einen Satz von Wandlersteuerungsparametern (342) anhand des Behandlungsplans derartig zu berechnen (102, 208), dass ein Schätzwert für die Ultraschallintensität (900) in der geschützten Zone unterhalb eines vorgegebenen Schwellenwerts liegt, wobei der Satz von Wandlersteuerungsparametern das Schalten von elektrischer Energie für jedes der Vielzahl von Wandlerelementen spezifiziert, **dadurch gekennzeichnet, dass**
- der Schätzwert für die Ultraschallintensität unter Verwendung einer inkohärenten Summe des durch jedes der Vielzahl von Wandlerelementen erzeugten Ultraschalldrucks berechnet wird.

2. Medizinisches Instrument nach Anspruch 1, wobei die inkohärente Summe des durch jedes der Vielzahl von Wandlerelementen erzeugten Ultraschalldrucks mit einem Kohärenzfaktor (516, 1000) multipliziert wird, um den Schätzwert für die Ultraschallintensität zu berechnen.

3. Medizinisches Instrument nach Anspruch 2, wobei der Kohärenzfaktor räumlich abhängig ist.

4. Medizinisches Instrument nach Anspruch 3, wobei das medizinische Instrument weiterhin ein medizinisches Bildgebungssystem (502, 602) zum Erfassen medizinischer Bilddaten (510) innerhalb einer Bildgebungszone (504) umfasst, wobei die Ausführung der Anweisungen den Prozessor weiterhin veranlasst, die medizinischen Bilddaten zu erfassen (202), wobei die geschützte Zone innerhalb der Bildgebungszone liegt, und wobei der Satz von Wandlersteuerungsparametern zumindest teilweise unter Verwendung der medizinischen Bilddaten berechnet wird.

5. Medizinisches Instrument nach Anspruch 4, wobei die Ausführung der Anweisungen den Prozessor weiterhin veranlasst:
- eine Bildsegmentierung (514) anhand der medizinischen Bilddaten zu berechnen (204), wobei die Bildsegmentierung Gewebetypen innerhalb des Patienten identifiziert; und
- den Kohärenzfaktor zumindest teilweise anhand der Bildsegmentierung zu berechnen (206).

6. Medizinisches Instrument nach Anspruch 4 oder 5, wobei das medizinische Bildgebungssystem eines der folgenden Systeme ist: ein Computertomographie-System, ein Magnetresonanz-Bildgebungssystem (602) und ein diagnostisches Ultraschallsystem.

7. Medizinisches Instrument nach einem der Ansprüche 3 bis 6, wobei der Kohärenzfaktor zumindest teilweise anhand einer kohärenten Summe (800) des durch jedes der Vielzahl von Wandlerelementen erzeugten Ultraschalldrucks berechnet wird.

8. Medizinisches Instrument nach Anspruch 7, wobei der Ultraschallwandler einen elektronisch einstellbaren Fokus zum Fokussieren von Ultraschallenergie in eine Zielzone hat, wobei das mit hochintensivem fokussiertem Ultraschall arbeitende System betriebsfähig ist, um den elektronisch einstellbaren Fokus zu steuern, indem es die Phase der elektrischen Energie für jedes der Vielzahl von Wandlerelementen steuert, wobei die Zielzone ein Pfad ist, wobei die Ausführung der Anweisungen den Prozessor weiterhin veranlasst:
- einen Satz von zeitabhängigen Steuerungsphasen zu berechnen, wobei die zeitabhängigen Steuerungsphasen die Phase der jedem der Vielzahl von Wandlerelementen zugeführten elektrischen Energie als eine Funktion der Zeit spezifiziert, so dass der elektronisch einstellbare Fokus dem Pfad folgt; und
- die kohärente Summe zumindest teilweise unter Verwendung des Satzes zeitabhängiger Steuerungsphasen zu berechnen.

9. Medizinisches Instrument nach einem der vorhergehenden Ansprüche, wobei der Satz von Wandlersteuerungsparametern weiterhin eines der folgenden Elemente umfasst: Phase der jedem der Vielzahl von Wandlerelementen zugeführten elektrischen Energie, Amplitude der jedem der Vielzahl von Wandlerelementen zugeführten elektrischen Energie, Energieniveau der jedem der Vielzahl von Wandlerelementen zugeführten elektrischen Energie, Wechselfrequenz der jedem der Vielzahl von Wandlerelementen zugeführten elektrischen Energie, Dauer der jedem der Vielzahl von Wandlerelementen zugeführten elektrischen Energie, Beschallungstrajektorie, und Kombinationen hiervon.

10. Medizinisches Instrument nach einem der vorhergehenden Ansprüche, wobei der Satz von Wandlerelementparametern berechnet wird, indem das Ein- und Ausschalten von Kombinationen der Vielzahl von Wandlerelementen simuliert wird.

11. Medizinisches Instrument nach einem der vorhergehenden Ansprüche, wobei der Satz von Phasen berechnet wird, indem ein kombinatorisches Optimierungsproblem gelöst wird.

12. Medizinisches Instrument nach einem der vorhergehenden Ansprüche, wobei die Ausführung der Anweisungen den Prozessor weiterhin veranlasst, mindestens die geschützte Zone als mehrere Regionen zu modellieren, und wobei der Satz von Wandlerelementzuständen unter Anwendung eines linearen Programmierproblems für die mehreren Regionen gelöst wird.

13. Medizinisches Instrument nach einem der vorhergehenden Ansprüche, wobei die geschützte Zone mehrere unterbrochene Volumina umfasst.

14. Computerprogrammprodukt mit maschinenausführbaren Anweisungen (350, 352, 354, 520, 522, 524) zur Ausführung durch einen Prozessor (328), der ein medizinisches Instrument (300, 500, 600) steuert, wobei das medizinische Instrument ein mit hochintensivem fokussiertem Ultraschall arbeitendes System (302) umfasst, das einen Ultraschallwandler (306) umfasst, wobei der Ultraschallwandler eine Vielzahl von Wandlerelementen (400, 402, 404, 406, 408) umfasst, wobei das mit hochintensivem fokussiertem Ultraschall arbeitende System betriebsfähig ist, um die Zuführung von elektrischer Energie zu jedem der Vielzahl von Wandlerelementen ein- und auszuschalten, wobei die Ausführung der Anweisungen den Prozessor veranlasst:
- einen Behandlungsplan (340_{[E1]}), der eine geschützte Zone (322) innerhalb eines Patienten (301) spezifiziert, zu empfangen (100, 200);
- einen Satz von Wandlersteuerungsparametern (342) anhand des Behandlungsplans derartig zu berechnen (102, 208), dass ein Schätzwert für die Ultraschallintensität (900) in der geschützten Zone unterhalb eines vorgegebenen Schwellenwerts liegt, wobei der Satz von Wandlerelementzuständen das Schalten von elektrischer Energie für jedes der Vielzahl von Wandlerelementen spezifiziert, wobei der Schätzwert für die Ultraschallintensität unter Verwendung einer inkohärenten Summe des durch jedes der Vielzahl von Wandlerelementen erzeugten Ultraschalldrucks berechnet wird.

15. Verfahren zum Betrieb eines medizinischen Instruments (300, 500, 600), das ein mit hochintensivem fokussiertem Ultraschall arbeitendes System (302) umfasst, wobei das mit hochintensivem fokussiertem Ultraschall arbeitende System einen Ultraschallwandler (306) umfasst, wobei der Ultraschallwandler eine Vielzahl von Wandlerelementen (400, 402, 404, 406, 408) umfasst, wobei das mit hochintensivem fokussiertem Ultraschall arbeitende System betriebsfähig ist, um die Zuführung von elektrischer Energie zu jedem der Vielzahl von Wandlerelementen ein- und auszuschalten, wobei das Verfahren die folgenden Schritte umfasst:
- Empfangen (100, 200) eines Behandlungsplans (340), der eine geschützte Zone (322) innerhalb eines Patienten (301) spezifiziert;
- Berechnen (102, 208) eines Satzes von Wandlersteuerungsparametern (342) anhand des Behandlungsplans derartig, dass ein Schätzwert für die Ultraschallintensität (900) in der geschützten Zone unterhalb eines vorgegebenen Schwellenwerts liegt, wobei der Satz von Wandlerelementzuständen das Schalten von elektrischer Energie für jedes der Vielzahl von Wandlerelementen spezifiziert, wobei der Schätzwert für die Ultraschallintensität unter Verwendung einer inkohärenten Summe des durch jedes der Vielzahl von Wandlerelementen erzeugten Ultraschalldrucks berechnet wird.

## Revendications

1. Instrument médical (300, 500, 600) comprenant :
- un système ultrasonore focalisé de forte intensité (302) comprenant un transducteur ultrasonore (306) ; dans lequel le transducteur ultrasonore comprend de multiples éléments de transducteur (400, 402, 404, 406, 408), dans lequel le système ultrasonore focalisé de forte intensité est à même de connecter ou déconnecter la source d'énergie électrique sur chacun des multiples éléments du transducteur ;
- un processeur (328) pour commander l'instrument vertical ;
- une mémoire (334) contenant des instructions exécutables en machine (350, 352, 354, 520, 522, 524) ; dans lequel l'exécution des instructions amène le processeur à :
- recevoir (100, 200) un plan de traitement (340) spécifiant une zone protégée (322) dans un sujet (301) ;
- calculer (102, 208) un jeu de paramètres de commande de transducteur (342) utilisant le plan de traitement de sorte qu'une estimation d'intensité ultrasonore (900) dans la zone protégée se situe en dessous d'un seuil prédéterminé, dans lequel le jeu de paramètres de commande de transducteur spécifie la connexion d'énergie électrique à chacun des multiples éléments du transducteur, **caractérisé en ce que** :
- l'estimation d'intensité ultrasonore est calculée en utilisant une somme incohérente de la pression ultrasonore générée par chacun des multiples éléments du transducteur.

2. Instrument médical selon la revendication 1, dans lequel la somme incohérente de la pression ultrasonore générée par chacun des multiples éléments du transducteur est multipliée par un facteur de cohérence (516, 1000) pour calculer l'estimation d'intensité ultrasonore.

3. Instrument médical selon la revendication 2, dans lequel le facteur de cohérence est spatialement dépendant.

4. Instrument médical selon la revendication 3, dans lequel l'instrument médical comprend en outre un système d'imagerie médical (502, 602) pour acquérir des données d'images médicales (510) dans une zone d'imagerie (504), dans lequel l'exécution des instructions amène en outre le processeur à acquérir (202) les données d'images médicales, dans lequel la zone protégée se situe dans la zone d'imagerie et dans lequel le jeu de paramètres de commande du transducteur est calculé au moins en partie en utilisant les données d'images médicales.

5. Instrument médical selon la revendication 4, dans lequel l'exécution des instructions amène en outre le processeur à :
- calculer (204) une segmentation d'image (514) en utilisant les données d'images médicales, dans lequel la segmentation d'image identifie les types de tissus au sein du sujet ; et
- calculer (206) le facteur de cohérence au moins en partie en utilisant la segmentation d'image.

6. Instrument médical selon la revendication 4 ou la revendication 5, dans lequel le système d'imagerie médical est l'un quelconque des suivants : système de tomographie à calculateur intégré, système d'imagerie à résonance magnétique (602) et système ultrasonore de diagnostic.

7. Instrument médical selon l'une quelconque des revendications 3 à 6, dans lequel le facteur de cohérence est calculé au moins en partie en utilisant une somme cohérente (800) de la pression ultrasonore générée par chacun des multiples éléments du transducteur.

8. Instrument médical selon la revendication 7, dans lequel le transducteur ultrasonore a un foyer réglable par voie électronique pour focaliser l'énergie ultrasonore appliquée dans une zone cible, dans lequel le système ultrasonore focalisé de forte intensité est à même de commander le foyer réglable par voie électronique en commandant la phase de l'énergie électrique appliquée à chacun des multiples éléments du transducteur, dans lequel la zone cible est un trajet, dans lequel l'exécution des instructions amène en outre le processeur à :
- calculer un jeu de phases de commande en fonction du temps, dans lequel les phases de commande en fonction du temps spécifient la phase de l'énergie électrique fournie à chacun des multiples éléments du transducteur en fonction du temps de sorte que le foyer réglable par voie électronique suive le trajet ; et
- calculer la somme cohérente au moins en partie en utilisant le jeu de phases de commande en fonction du temps.

9. Instrument médical selon l'une quelconque des revendications précédentes, dans lequel le jeu de paramètres de commande du transducteur comprend en outre l'un quelconque des paramètres suivants : phase de l'énergie électrique fournie à chacun des multiples éléments du transducteur, amplitude de l'énergie électrique fournie à chacun des multiples éléments du transducteur, niveau d'énergie de l'énergie électrique fournie à chacun des multiples éléments du transducteur, fréquence alternative de l'énergie électrique appliquée à chacun des multiples éléments du transducteur, durée de l'énergie électrique fournie à chacun des multiples éléments du transducteur, trajectoire de sonification et leurs combinaisons.

10. Instrument médical selon l'une quelconque des revendications précédentes, dans lequel le jeu de paramètres d'éléments du transducteur est calculé en simulant la connexion et la déconnexion de combinaisons des multiples éléments du transducteur.

11. Instrument médical selon l'une quelconque des revendications précédentes, dans lequel le jeu de phases est calculé en résolvant un problème d'optimisation combinatoire.

12. Instrument médical selon l'une quelconque des revendications précédentes, dans lequel l'exécution des instructions amène en outre le processeur à modéliser au moins la zone protégée sous la forme de régions multiples et dans lequel le jeu d'états des éléments du transducteur est résolu en utilisant un problème de programmation linéaire pour les multiples régions.

13. Instrument médical selon l'une quelconque des revendications précédentes, dans lequel la zone protégée comprend de multiples volumes déconnectés.

14. Produit de programmation informatique comprenant des instructions exécutables en machine (350, 352, 354, 520, 522, 524) pour exécution par un processeur (328) commandant un instrument médical (300, 500, 600), dans lequel l'instrument médical comprend un système ultrasonore focalisé de forte intensité (302) comprenant un transducteur ultrasonore (306) ; dans lequel le transducteur ultrasonore comprend de multiples éléments de transducteur (400, 402, 404, 406, 408), dans lequel le système ultrasonore focalisé de forte intensité est à même de connecter ou déconnecter la source d'énergie électrique sur chacun des multiples éléments du transducteur ; dans lequel l'exécution des instructions amène le processeur à :
- recevoir (100, 200) un plan de traitement (340_{[SP1]}) spécifiant une zone protégée (322) dans un sujet (301) ;
- calculer (102, 208) un jeu de paramètres de commande de transducteur (342) utilisant le plan de traitement de sorte qu'une estimation d'intensité ultrasonore (900) dans la zone protégée se situe en dessous d'un seuil prédéterminé, dans lequel le jeu de paramètres de commande de transducteur spécifie la connexion d'énergie électrique à chacun des multiples éléments du transducteur, dans lequel l'estimation d'intensité ultrasonore est calculée en utilisant une somme incohérente de la pression ultrasonore générée par chacun des multiples éléments du transducteur.

15. Procédé de fonctionnement d'un instrument médical (300, 500, 600) comprenant un système ultrasonore focalisé de forte intensité (302), dans lequel le système ultrasonore focalisé de forte intensité comprend un transducteur ultrasonore (306) ; dans lequel le transducteur ultrasonore comprend de multiples éléments de transducteur (400, 402, 404, 406, 408), dans lequel le système ultrasonore focalisé de forte intensité est à même de connecter ou déconnecter la source d'énergie électrique sur chacun des multiples éléments du transducteur ; dans lequel le procédé comprend les étapes consistant à :
- recevoir (100, 200) un plan de traitement (340) spécifiant une zone protégée (322) dans un sujet (301) ;
- calculer (102, 208) un jeu de paramètres de commande de transducteur (342) utilisant le plan de traitement de sorte qu'une estimation d'intensité ultrasonore (900) dans la zone protégée se situe en dessous d'un seuil prédéterminé, dans lequel le jeu de paramètres de commande de transducteur spécifie la connexion d'énergie électrique à chacun des multiples éléments du transducteur, dans lequel l'estimation d'intensité ultrasonore est calculée en utilisant une somme incohérente de la pression ultrasonore générée par chacun des multiples éléments du transducteur.
